(19) Europäisches Patentamt

European Patent Office

Office européen des brevets

(11) Veröffentlichungsnummer: **0 295 550 B1**

(12) **EUROPÄISCHE PATENTSCHRIFT**

(45) Veröffentlichungstag der Patentschrift: **27.11.91**

(21) Anmeldenummer: **88109087.2**

(22) Anmeldetag: **08.06.88**

(51) Int. Cl.5: **C07C 229/08**, C07C 227/08, C07C 227/32

(54) **Verfahren zur Herstellung von D- oder L-Alanin hoher Enantiomerenreinheit.**

(30) Priorität: **13.06.87 DE 3719872**

(43) Veröffentlichungstag der Anmeldung:
**21.12.88 Patentblatt 88/51**

(45) Bekanntmachung des Hinweises auf die
Patenterteilung:
**27.11.91 Patentblatt 91/48**

(84) Benannte Vertragsstaaten:
**AT BE CH DE ES FR GB IT LI NL**

(56) Entgegenhaltungen:

**BERICHTE DER DEUTSCHEN CHEMISCHEN GESELLSCHAFT, Jahrgang 51, Band II, 1918, Kommissionsverlag von R. Friedländer & Sohn**

**BERICHTE DER DEUTSCHEN CHEMISCHEN GESELLSCHAFT, Jahrgang 40, Band I, 1907, Kommissionsverlag von R. Friedländer & Sohn**

**CHEMICAL ABSTRACTS, Band 95, Nr. 5, 3rd August 1981, Seite 811, Abstract Nr. 43613f, Columbus, Ohio, USA; Y. OGATA et al.:**

**"Preparation of DL-alanine by the reaction of Dl-Alpha-chloropropionic acid with aqueous ammonia under pressure, & KENKYU HOKO-KU - ASAHI GARASU KOGYO GIJUTSU SHO-REIKAI 1980, (36), 219-24**

(73) Patentinhaber: **BASF Aktiengesellschaft Carl-Bosch-Strasse 38 W-6700 Ludwigshafen(DE)**

(72) Erfinder: **Paul, Axel, Dr. Kaefertaler Strasse 38 W-6800 Mannheim 1(DE)**
Erfinder: **Peter, Tonne, Dr. Triftbrunnenweg 63 W-6730 Neustadt(DE)**
Erfinder: **Roske, Eckhard Hillensheimer Strasse 4 W-6700 Ludwigshafen(DE)**
Erfinder: **Siegel, Hardo, Dr. Hans-Purrmann-Allee 25 W-6720 Speyer(DE)**

## Beschreibung

Die Erfindung betrifft ein Verfahren zur Herstellung von D- oder L-Alanin hoher Enantiomerenreinheit, welches dadurch gekennzeichnet ist, daß man eine L- oder D-Chlorpropionsäure hoher Enantiomerenreinheit mit Ammoniak oder Ammoniak liefernden Verbindungen in Wasser oder Wasser/Alkohol-Gemischen bei Temperaturen zwischen 50 und 110° C umsetzt.

Die Herstellung von racemischen D,L-Alanin aus Halogenpropionsäuren ist schon mehrfach beschrieben worden. J. Ogata et al. Bull. Chem. Soc. Jpn., 54, 3605 (1981) beschreiben, daß sich D,L-Chlorpropionsäure durch Umsetzen mit einem Überschuß NH₃ in Wasser bei 70° C in D,L-Alanin überführen läßt.

Weiterhin ist bekannt, daß sich z.B. β-Alanin aus β-Brompropionsäure darstellen läßt (N.L. Wender, J. Am. Chem. Soc. 71, 375 (1949)). Dabei wird die β-Brompropionsäure mit mindestens stöchiometrischen Mengen Urotropin umgesetzt. Anschließend wird das entstandene quartäre Ammoniumsalz mit conc. HCl zu β-Alanin Hydrochlorid gespalten.

In der US-A 3 190 914 wird des weiteren ein Verfahren beschrieben, das besonders zur Herstellung von Glycin aus Chloressigsäure geeignet ist. Dabei werden mindestens stöchiometrische Mengen Urotropin bezüglich der Chloressigsäure und zusätzlich ein Überschuß an NH₃ in Wasser eingesetzt.

Der russischen Patentschrift SU 763 329 zufolge wird u.a. D,L-Alanin aus D,L-Chlorpropionsäure durch Umsetzung mit NH₃/H₂O unter Zusatz von Melamin, Urotropin oder Cyanursäure (üblicherweise 15 % bezogen auf D,L-Chlorpropionsäure) erhalten.

Die beiden letztgenannten Verfahren kommen ohne saure Hydrolyse aus, es fällt direkt die Aminosäure an.

Diese für die Umsetzung von racemischen Chlorcarbonsäuren bekannten Verfahren galten offensichtlich als nicht auf optisch aktives Ausgangsmaterial ohne Racemisierungsgefahr übertragbar, da alle für die Herstellung von D-Alanin beschriebene Verfahren aufwendigere und teurere Wege gehen.

Dies sind, neben der fermentativen Herstellung von D-Alanin und verschiedenen enzymatischen und Kristallisations-Racematspaltungen aus racemischem Alanin, mehrere relativ aufwendige enantioselektive Synthesen zur Darstellung von D-Alanin, die zudem Produkte von teilweise unbefriedigender Enantiomeren-Reinheit liefern. Aus Nature 1950 - (166), Seite 178-179 ist beispielsweise die Umsetzung von L-α-Brompropionsäure mit NaN₃ und anschließender Hydrierung bekannt. Ferner führt die enantioselektive aminierende Hydrierung von Brenztraubensäure bzw. Acetamidoacrylsäure gemäß Journal of Organomet. Chem. 1978 (150), Seite C14-C16 zu D-Alanin. Nach den Angaben der GB-A 2 100 264 kann man ferner aus dem Mesylat von L-Milchsäureestern durch Umsetzung mit Benzylamin, Hydrierung und Verseifung D-Alanin gewinnen. Schließlich führt die enantioselektive enzymatische Spaltung von D,L-Alaninamid zu D-Alanin und L-Alaninamid gemäß der NL-A 84/3093.

Nach den Angaben von Ber. 51, 1315-1322 (1918) entsteht bei der Umsetzung von L-Chlorpropionsäure mit Ammoniak in wäßriger Lösung bei Raumtemperatur während einer Reaktionsdauer von 14 Tagen linksdrehendes L-Alanin. Eine Angabe zur Enantiomerenreinheit und zur Ausbeute des erhaltenen L-Alanins fehlt.

Es wurde nun ein Verfahren zur Herstellung von D- oder L-Alanin hoher Enantiomerenreinheit gefunden, welches dadurch gekennzeichnet ist, daß man eine L- oder D-Chlorpropionsäure hoher Enantiomerenreinheit mit Ammoniak oder Ammoniak liefernden Verbindungen in Wasser oder Wasser/Alkohol-Gemischen bei Temperaturen zwischen 50 und 110° C umsetzt.

Die Reaktion eignet sich gleichermaßen zur Herstellung von D-Alanin aus L-Chlorpropionsäure als auch von L-Alanin aus D-Chlorpropionsäure. Insbesondere wird das Verfahren zur Herstellung von D-Alanin aus L-Chlorpropionsäure verwendet.

Zweckmäßig wendet man das Ammoniak bzw. das Ammoniak liefernde Reagens in der insgesamt mindestens zweifach molaren Menge, bezogen auf die Chlorpropionsäure, an. Dabei wird in der Regel das Ammoniak in dem Maße zugegeben, daß ein pH-Wertbereich von pH 6 bis 9, vorzugsweise 6,5 bis 7,5, eingehalten wird.

Als Ammoniak liefernde Verbindung kommt beispielsweise Urotropin in Betracht. Das Urotropin kann vorteilhaft in z.B. 10 bis 100 Mol.%, bevorzugt 15 bis 30 Mol.%, bezogen auf L-Chlorpropionsäure verwendet werden. Größere Mengen sind jedoch nicht ausgeschlossen.

Als Alkohole sind niedermolekulare, vorzugsweise wasserlösliche Alkohole, z.B. Methanol, Ethanol, Isopropanol, n-Propanol, n-Butanol und Isobutanol zu nennen, wobei das Verhältnis Wasser zu Alkohol 60 zu 40 bis 100 zu 0 Vol.% betragen kann.

Anstelle der freien Chlorpropionsäure kann man auch von den entsprechenden Alkali- oder Erdalkalisalzen ausgehen, wobei es sich jedoch empfiehlt, durch Umsetzung mit Salzsäure die freie Säure zumindest teilweise in Freiheit zu setzen, um die Bildung von Nebenprodukten, wie 2,2'-Iminodipropionsäure zu verhindern. Selbstverständlich kann man auch von dem Ammoniumsalz der Chlorpropionsäure ausgehen. In diesem Fall ist für die Chlorsubstitution nur die mindestens einfach molare Menge Ammoniak erforderlich.

Im einzelnen geht man bei der erfindungsgemäßen Umsetzung im allgemeinen so vor: Man legt die L-Chlorpropionsäure als wäßrige, gegebenenfalls salzhaltige, z.B. NaCl- oder $Na_2SO_4$-haltige Lösung vor. Die Wassermenge wird üblicherweise so gewählt, daß gerade eine homogene Lösung vorliegt. Es ist aber auch möglich, mehr Wasser zu verwenden. Anstelle von Wasser können auch Wasser/Alkohol-Gemische eingesetzt werden. Durch Zugabe von verdünnter Ammoniaklösung, z.B. 25 %igem Ammoniakwasser wird ein pH-Bereich von etwa 6 bis 7 eingestellt und dann wird die gewünschte Menge Urotropin hinzugefügt. Natürlich ist die Zugabereihenfolge Urotropin/Ammoniaklösung vertauschbar. Anschließend wird das Reaktionsgemisch auf die Reaktionstemperatur von 50 bis 110, insbesondere 50 bis 90, vorzugsweise 60 bis 70°C, erhitzt, wobei der pH-Wert der Lösung durch Zugabe von verdünnter Ammoniaklösung bei 6 bis 9, insbesondere 6,5 bis 7,5, vorzugsweise 6,8 bis 7,2 gehalten wird. Die Reaktionszeit beträgt bei einem Anteil von 20 Mol.% Urotropin, bezogen auf L-Chlorpropionsäure ca. 4 Stunden. Durch höhere Urotropinmengen kann die Reaktionszeit herabgesetzt werden.

Bei Umsetzung in Abwesenheit von Urotropin kann man z.B. wie folgt vorgehen: Man löst die L-Chlorpropionsäure oder ihr Alkali- oder Erdalkalisalz in der 5- bis 15-fachen, vorzugsweise 10-fach molaren Menge Ammoniakwasser (z.B. 25 %ig) und rührt das Reaktionsgemisch in einem druckfesten Gefäß unter Eigendruck bei der Reaktionstemperatur. Bei einer Temperatur von 70°C ist die Umsetzung nach ca. 5 Stunden beendet.

Die Aufarbeitung der Reaktionsgemische kann in an sich üblicher Weise erfolgen. Beispielsweise kann man die im Reaktionsaustrag gelösten anorganischen Salze auf den gewünschten Prozentsatz abreichern, wozu sich insbesondere die bekannten Elektrodialyseverfahren eignen. Gegebenenfalls im Reaktionsgemisch vorhandenes Urotropin kann z.B. durch Hydrolyse, durch Salzbildung mit organischen Säuren oder insbesondere Mineralsäuren oder durch Reduktion mit Ameisensäure beseitigt werden. Die Isolierung des D-Alanins kann vorteilhaft durch selektive Kristallisation nach Einengen der Lösung vorgenommen werden.

Die als Ausgangsmaterial verwendeten optisch aktiven Chlorpropionsäuren sind aus den entsprechenden, enzymatisch hergestellten optisch aktiven Milchsäuren in technischen Mengen zugänglich (s. hierzu EP-A 00 56 981).

Beispiel 1

10,2 g (0,094 mol) L-Chlorpropionsäure (96 % ee) wurden zusammen mit 150 g 25 %iger $NH_3$-Lösung (2,2 mol) 5 h bei 70°C in einem Autoklaven gerührt. Der Austrag wurde mit conc. HCl auf pH 6,1 eingestellt und durch Elektrodialyse entsalzt. Anschließende Kristallisation durch Einengen der wässrigen Lösung lieferte 5,8 g (0,065 mol) reines D-Alanin (70 % Ausbeute, ee > 98 %).

Beispiel 2

108,5 g (1 mol) L-Chlorpropionsäure (96 % ee) wurden in 160 ml Wasser gelöst, mit 28 g (0,2 mol) Urotropin und 66,8 g (0,98 mol) 25 %iger wäßriger $NH_3$-Lösung versetzt und auf 65°C erwärmt. Es wurden 67,5 g (1,0 mol) 25 %iger $NH_3$-Lösung so zudosiert, daß der pH-Wert des Reaktionsgemisches stets bei 6,9 lag. Die Reaktionszeit betrug ca. 4 h. Der Austrag wurde mit HCl auf pH 6,1 gestellt und mit 250 ml Wasser versetzt, so daß eine homogene Lösung erhalten wurde. Diese wurde durch Elektrodialyse bis auf einen Chlorid-Gehalt von 0,04 % entsalzt. Anschließende Kristallisation lieferte 65 g D-Alanin (0,73 mol, 73 % Ausbeute, ee > 98 %).

Beispiel 3

Eine Lösung von 130,5 g (1 mol) Na-L-Chlorpropionat (ee 96 %), gewonnen durch Verseifung eines L-Chlorpropionsäureesters, wurde mit 96 g (1 mol) 38 %iger HCl versetzt. Das Gemisch wurde mit 28 g (0,2 mol) Urotropin und 67,5 g (1 mol) 25 %iger wäßriger $NH_3$-Lösung versetzt, auf 65°C erwärmt und dann weiter entsprechend Beispiel 2 umgesetzt. Es wurden 64 g (0,72 mol) D-Alanin erhalten (72 % Ausbeute, ee > 98 %).

**Patentansprüche**

1. Verfahren zur Herstellung von D- oder L-Alanin hoher Enantiomerenreinheit, dadurch gekennzeichnet, daß man eine L- oder D-Chlorpropionsäure hoher Enantiomerenreinheit mit Ammoniak oder Ammoniak liefernden Verbindungen in Wasser oder Wasser/Alkohol-Gemischen bei Temperaturen zwischen 50 und 110°C umsetzt.

2. Verfahren nach Anspruch 1, dadurch gekennzeichnet, daß man als Ammoniak liefernde Verbindung Urotropin verwendet.

3. Verfahren nach Anspruch 1, dadurch gekennzeichnet, daß man Gemische aus Ammoniak und Urotropin verwendet.

4. Verfahren nach Anspruch 1, dadurch gekennzeichnet, daß man insgesamt mindestens 2 mol Ammoniak und Ammoniak liefernde Verbindungen pro mol Chlorpropionsäure anwendet.

5. Verfahren nach Anspruch 1, dadurch gekennzeichnet, daß man die Umsetzung bei 50 bis 90°C und einem pH-Wert zwischen 6 und 9 durchführt.

6. Verfahren nach Anspruch 1, dadurch gekennzeichnet, daß man D-Alanin aus L-Chlorpropionsäure herstellt.

7. Verfahren nach Anspruch 1, dadurch gekennzeichnet, daß man L-Alanin aus D-Chlorpropionsäure herstellt.

## Claims

1. A process for the preparation of D- or L-alanine of high enantiomeric purity, wherein an L- or D-chloropropionic acid of high enantiomeric purity is reacted with ammonia or an ammonia-donating compound in water or a water/alcohol mixture at from 50 to 110°C.

2. A process as claimed in claim 1, wherein the ammonia-donating compound used is urotropine.

3. A process as claimed in claim 1, wherein a mixture of ammonia and urotropine is used.

4. A process as claimed in claim 1, wherein the total amount of ammonia or ammonia-donating compound used is not less than 2 moles per mole of chloropropionic acid.

5. A process as claimed in claim 1, wherein the reaction is carried out at from 50 to 90°C and at a pH of from 6 to 9.

6. A process as claimed in claim 1, wherein D-alanine is prepared from L-chloropropionic acid.

7. A process as claimed in claim 1, wherein L-alanine is prepared from D-chloropropionic acid.

## Revendications

1. Procédé de préparation de D- ou L-alanine de haute pureté énantiomère, caractérisé en ce qu'on fait réagir un acide L- ou D-chloropropionique de haute pureté énantiomère avec de l'ammoniac ou des composés fournissant de l'ammoniac, dans de l'eau ou dans des mélanges eau/alcool, à des températures comprises entre 50 et 110°C.

2. Procédé selon la revendication 1, caractérisé en ce qu'on utilise de l'urotropine comme composé fournissant de l'ammoniac.

3. Procédé selon la revendication 1, caractérise en ce qu'on utilise des mélanges d'ammoniac et d'urotropine.

4. Procédé selon la revendication 1, caractérisé en ce qu'on utilise au total au moins 2 moles d'ammoniac et de composés fournissant de l'ammoniac par mole d'acide chloropropionique.

5. Procédé selon la revendication 1, caractérisé en ce qu'on conduit la réaction à une température de 50 à 90°C et à un pH compris entre 6 et 9.

6. Procédé selon la revendication 1, caractérisé en ce qu'on prépare de la D-alanine à partir d'acide L-chloropropionique.

7. Procédé selon le revendication 1, caractérisé en ce qu'on prépare de la L-alanine à partir d'acide D-chloropropionique.